# EUROPEAN PATENT APPLICATION

(11) **EP 2 290 570 A2**
(43) Date of publication of application: **02.03.2011**
(21) Application number: 10008207.2
(22) Date of filing: 06.08.2010
(51) Int. Cl.: G06F 19/00

(54) **System and method for transcribing medical information**

(30) Priority: 26.08.2009 US 547661
(71) Applicant: Perry Johnson and Associates, Inc., Henderson NV 89012 (US)
(72) Inventor: Johnson, Perry L., Bloomfield Hills. Michigan 48304 (US); Carrillo, Manuel A., Dearborn,Michigan 48128 (US); Uddin, Mohammed Rohim, Bangalore 45 (IN)
(74) Representative: GPI & Associés

(57) **Abstract**

A method for transcribing medical information may include receiving input assigning each of a plurality of user profiles to at least one of a plurality of work pools, receiving a plurality of jobs each including demographic information, a priority indicator, and a digital audio file, assigning each of the jobs to one of the plurality of work pools based on the job's priority indicator and demographic information, distributing each of the assigned jobs to one of the assigned user profiles active within the assigned job's work pool, receiving, for each of the assigned jobs, a transcription of the job's digital audio file, generating, for a selected user profile, quality metrics indicative of an accuracy of transcriptions input, and outputting a visual representation of the quality metrics.

## Description

### BACKGROUND

United States Patent Number 7,031,998 to Archbold discloses a system that includes one or more "HOME"s where there is a (1) "DAD" computer and software system for human User interaction to initiate Jobs (Transcription, Translation, Data Entry, and Transaction Creation) and to utilize voice-mail through real-time voice and tone signal input and (2) "HIS" computer and software system for keeping the databases used to process Jobs and for keeping the statistical records of jobs for analysis, general ledger, payroll and billing. In addition, the system includes one or more "MOM"s where there is a software system server on a computer set on the backbone of a global communication network (e.g. Internet) that (1) receives Job packets from its HOMEs, (2) selects Scribes for work (e.g. transcription), (3) generates messages in the form of E-Mail and Voice-Mail to specified users for information or action, (4) updates the status of the Job Record and transfers that updated Record to a SUPERMOM, and (5) implements all the details of job processing steps and logic that has been controlled by Human Supervisors. The system may further include a "SUPERMOM" where there is a software system server on a computer set on the backbone of the global computer network (e.g. Internet) that receives Job Packets from MOMs where portions of the job are spread over two or more MOMs; SUPERMOM directs and transfers that job data; receives Job Record updates and maintains a Site for on-line internet job tracking inquiries; SUPERMOM selects Scribes for Jobs that are not able to find an available Scribe within their own MOM's HOMEs, collects the statistical and financial data from all the HOMEs, and provides general top level management decision making information.

### SUMMARY

A system for transcribing medical information includes one or more computers configured to receive input assigning each of a plurality of user profiles to at least one of a plurality of work pools based on user profile criteria, to receive a plurality of jobs each including demographic information, a priority indicator, and a digital audio file of patient medical information, and to assign each of the jobs to one of the plurality of work pools based on the job's priority indicator and demographic information. The one or more computers are further configured to distribute each of the assigned jobs to one of the assigned user profiles active within the assigned job's work pool based on the assigned job's priority indicator, to receive, for each of the assigned jobs, a transcription of the job's digital audio file input by a user associated with the job's assigned user profile, to generate, for a selected user profile, quality metrics indicative of an accuracy of transcriptions input by the user associated with the selected user profile, and to output a visual representation of the quality metrics.

While example embodiments in accordance with the invention are illustrated and disclosed, such disclosure should not be construed to limit the invention. It is anticipated that various modifications and alternative designs may be made without departing from the scope of the invention.

### BRIEF DESCRIPTION OF THE DRAWINGS

FIGURE 1 is a block diagram of an embodiment of a medical transcription system.
FIGURE 2 is a block diagram of the application server of Figure 1 and shows user profiles being assigned to work pools.
FIGURE 3 is another block diagram of the application server of Figure 1 and shows jobs being received by the application server.
FIGURE 4 is a block diagram of the database server of Figure 1 and shows records within a database.
FIGURE 5 is another block diagram of the application server of Figure 1 and shows jobs being assigned to work pools.
FIGURE 6 is another block diagram of the application server of Figure 1 and shows jobs being assigned to active user profile 1 within work pool 1.
FIGURE 7 is another block diagram of the application server of Figure 1 and shows the assigned jobs of user profile 1 ordered by their turn-around time.
FIGURE 8 is another block diagram of the application server of Figure 1 and shows transcribed audio files being received by the application server.
FIGURE 9 is another block diagram of the application server of Figure 1 and shows quality indicators displayed by the application server.

### DETAILED DESCRIPTION

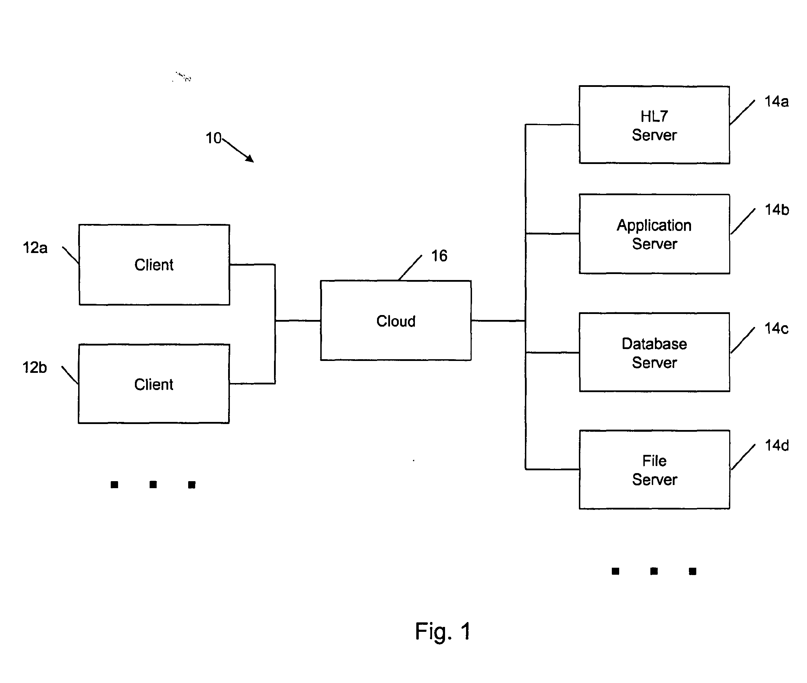

Referring now to Figure 1, an embodiment of a transcription system 10 may include a plurality of clients (computers, workstations, etc.) 12a, 12b, etc. (12n) and a plurality of servers (computers, workstations, etc.) 14a, 14b, etc. (14n) connected, in a known fashion, by a communications cloud 16 (e.g., Internet, etc.) Hence, the clients 12n may communicate with the serves 14n via the cloud 16; the servers 14n may communicate with each other and with the clients 12n via the cloud 16.

The clients 12n and servers 14n may be geographically remote from each other. In some embodiments, the clients 12n may be located in, for example, India, while the servers 14n may be located in the United States. In other embodiments, some of the clients 12n and servers 14n may be located in one country while other of the clients 12n and servers 14n may be located in another country. Other arrangements are also possible.

As will be discussed in detail below, the system 10 facilitates the transcription of medical information received in an audio format. Generally speaking, the servers 14n may receive, *inter alia,* audio files of medical information from hospitals, clinics, individuals, etc. These audio files may be managed and stored via the servers 14n. Medical transcriptionists may access the audio files stored on the servers 14n and input textual transcriptions, via applications on the clients 12n, to the servers 14n. Quality personnel may access the audio files and corresponding transcriptions stored on the servers 14n via the clients 12n to check the accuracy of the same. The servers 14n, based on these checks, may generate and output quality metrics associated with the transcriptions.

The servers 14n, in the embodiment of Figure 1, include an HL7 server 14a, application server 14b, database server 14c, and file server 14d. Other and/or different servers 14n may be used in other embodiments.

The HL7 server 14a receives information, via the cloud 16, from hospitals, clinics, etc. via an HL7 feed as known in the art. This information, as will be discussed in detail below, may include files of medical information, demographic information relating to the source of the information, and a priority assigned to the information. Information may also be received in any known/suitable fashion. For example, one or more of the servers 14n may be configured to receive medical information phoned in from the hospitals, clinics, etc. Other scenarios are also possible.

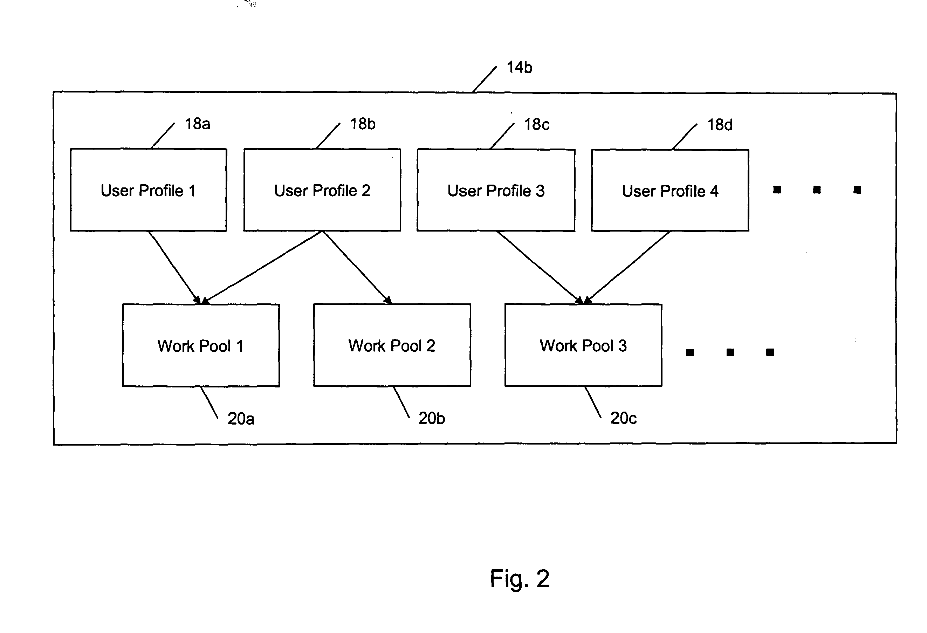

Referring now to Figure 2, a plurality of user profiles 18a, 18b, 18c, 18d, etc. (18n) and work pools 20a, 20b, 20c, etc. (20n) have been created within the application server 14b. As discussed below, each of the user profiles 18n may be assigned to one or more of the work pools 20n to facilitate the transcription of medical information.

Each of the user profiles 18n is associated with a medical transcriptionist. That is, information about a particular medical transcriptionist's proficiency, experience, etc. may define their user profile. The medical transcriptionist associated with the user profile 18a, for example, may have 2 years of experience and particular expertise in transcribing medical information from cardiologists. The medical transcriptionist associated with the user profile 18b, for example, may have 6 years experience and expertise in transcribing information from a wide array of medical specialists (including cardiologists) as well as family doctors. The medical transcriptionist associated with the user profile 18c, for example, may have 2 months experience and proficiency only enough to transcribe information from nurse practitioners.

Each of the work pools 20n has criteria that define the types of user profiles that may be assigned to it. Work pool 20a, for example, may require that its assigned user profiles have at least 3 years experience and expertise in transcribing medical information from cardiologists. Such a requirement may be necessary if medical information to be transcribed by members of the work pool 20a is received primarily from cardiology care providers. Hence, a work pool's criteria may be dictated by the type and/or source of medical information its members are to transcribe. The work pool 20b, for example, may require that its user profiles have at least 5 years experience. Other and/or different criteria may, of course, be used.

User profiles 18n may be assigned to the work pools 20n based on the criteria defining the work pools 20n. Using the examples described above, user profiles 18a, 18b have been assigned to work pool 20a because each of them has the requisite skills and experience required by the work pool 20a: each of the user profiles 18a, 18b has at least 3 years experience and expertise in transcribing medical information from cardiologists.

User profile 18b has been assigned to work pool 20b because the user profile 18b has at least 5 yeas experience, etc.

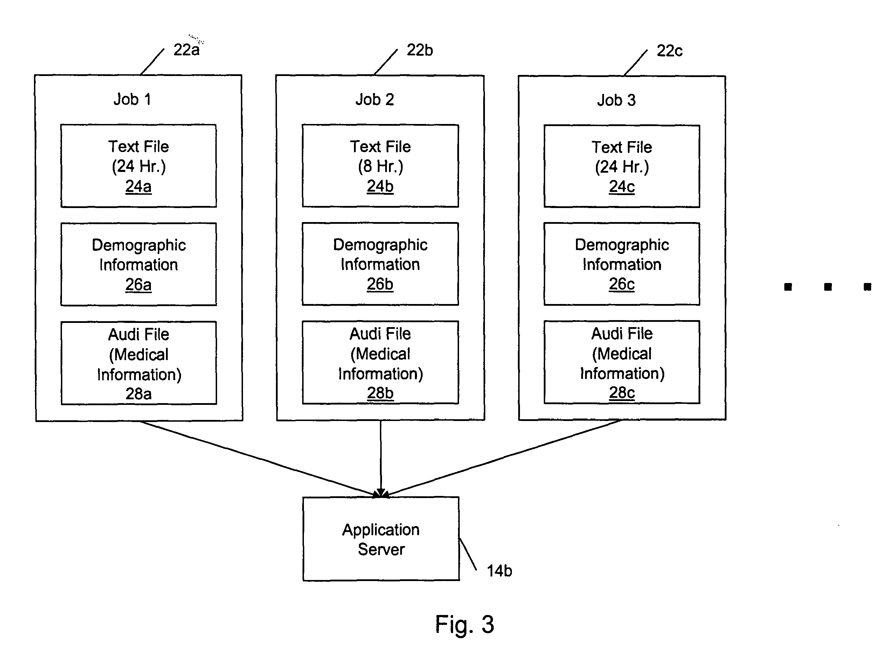

Referring now to Figure 3, information received from clinics, individuals, etc. is represented as jobs 22a, 22b, 22c, etc. (22n). Each of the jobs 22n includes, in the embodiment of Figure 3, a text file 24x, demographic information 26x, and audio file 28x. As will be discussed below, a data structure may be created within the database server 14c illustrated in Figure 1 to manage the information contained within the jobs 22n.

The text files 24x may include a turn-around time or other priority indicator (e.g., 8 hours, high, low, type of information within the text file 24x, etc.) The turn-around time, in this example, is the desired time by which the source of the information, such as a hospital, needs to have the transcribed medical information made available to them. Of course, the text files 24x may include any other suitable information.

The demographic information 26x may include particulars relating to a patient as well as the clinic, physician, etc. treating the patient. For example, the demographic information 26a may include the patient's name and age as well as the attending physician's name, etc. Of course, other and/or different information may be included.

The audio file 28x may include, as mentioned above, medical information about a patient to be transcribed. (Multiple jobs may spring from the same audio file if, for example, information from several patients is included is a single audio file.) The audio file 28x may be parsed from its job 22x and stored on the file server 14d illustrated in Figure 1.

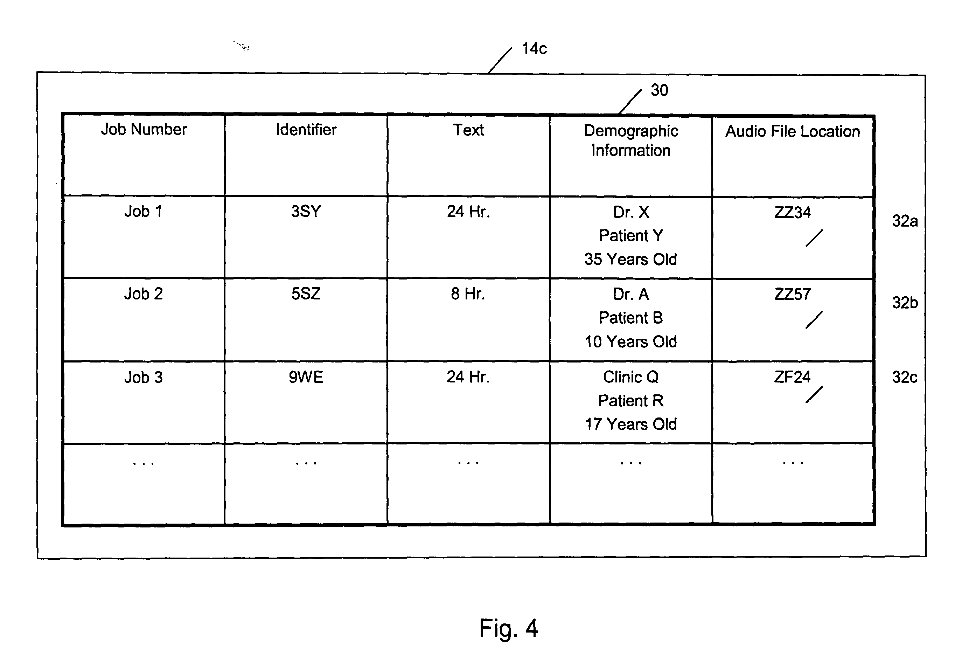

Referring now to Figure 4, the database server 14c has created, within a database 30, records 32a, 32b, 32c, etc. (32n) for each of the jobs 22n. (Each of the records 32n corresponds to one of the jobs 22n.) Each of the records 32n may include the number of the corresponding job 22x, an assigned identifier, text from the text file 24x of the job 22x, the demographic information 26x of the job 22x, and a location on the file server 14d illustrated in Figure 1 where the audio file 28x of the job 22x is stored, etc.

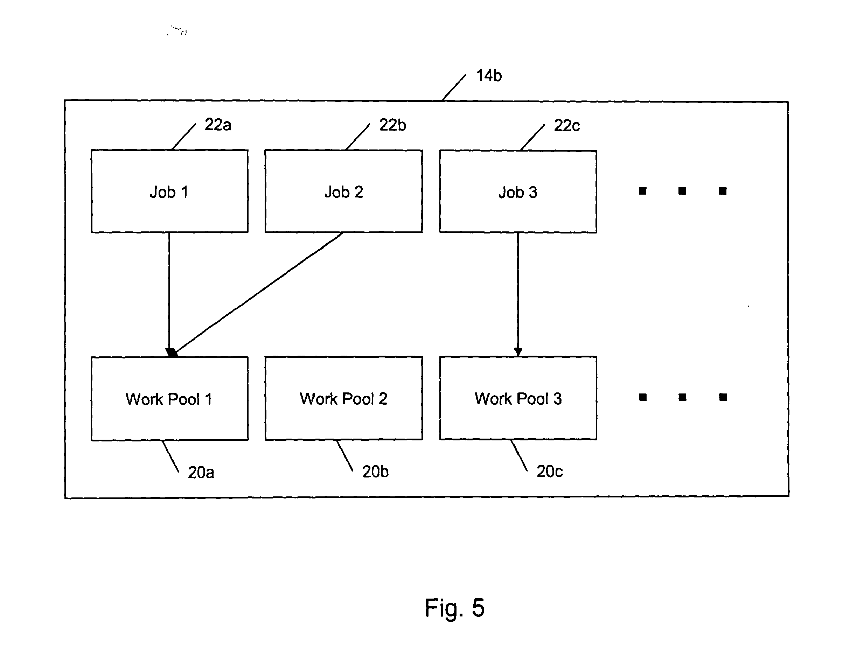

Referring now to Figure 5, the application server 14b may assign each of the jobs 22n to one of the work pools 20n based on, for example, the demographic information contained within each of the jobs 22n. For example, the jobs 22n from a particular clinic or practice group may be assigned exclusively to work pool 20a. The jobs 22n from a particular physician may be assigned to work pool 20c, etc. Other suitable methodologies, however, may be used to assign the jobs 22n to the work pools 20n.

As discussed above, the criteria that define which types of user profiles 18n are assigned to particular work pools 20n may depend on the source of the jobs 22n. Hence, the use of work pools 20n, in certain embodiments, may ensure that the jobs 22n requiring particular expertise are handled by user profiles 18n having that expertise.

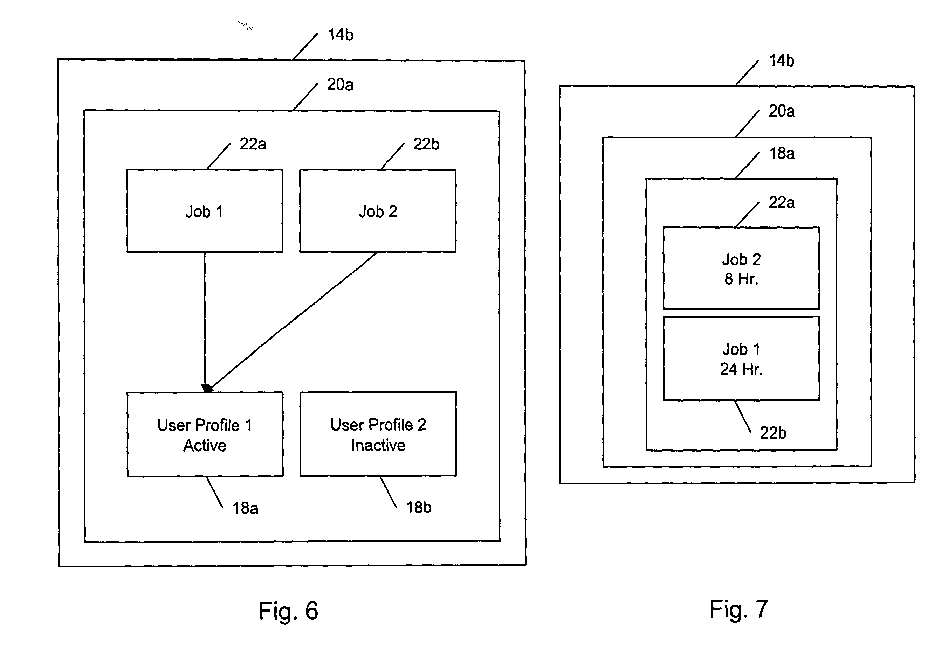

Referring now to Figure 6, the user profiles 18a, 18b assigned to the work pool 20a may be active or inactive. This depends on, for example, whether the medical transcriptionists associated with the user profiles 18a, 18b are logged in to the application server 14b via one of the clients 12n illustrated in Figure 1. In the example of Figure 6, the medical transcriptionist associated with the user profile 18a has logged in to the application server 14b. Hence, the user profile 18a is active. The medical transcriptionist associated with the user profile 18b has not logged in to the application server 14b. Hence, the user profile 18b is inactive.

The application server 14b may assign the jobs 22a, 22b within the work pool 20a to the active user profile 18a. (The inactive user profile 18b is not assigned any jobs as its corresponding medical transcriptionist is not logged in to the application server 14b.) If there are several active user profiles within a particular work pool, the application server 14b may distribute jobs between them using any suitable methodology. For example, jobs may be distributed equally among all the active profiles, etc.

Referring now to Figure 7, the application server 14b has ordered the jobs 22a, 22b assigned to the user profile 18a based on the priority indicator. The job 22b, in this example, has the higher priority (shorter turn-around time) and is thus ordered first. The medical transcriptionist associated with the user profile 18a is expected to work on the jobs 22a, 22b in the order they are assigned.

In certain embodiments, the medical transcriptionist associated with the user profile 18a may access the database 30 illustrated in Figure 4 to determine the location of the audio files 28a, 28b on the file server 14d illustrated in Figure 1, and retrieve them accordingly. In other embodiments, the audio files 28a, 28b may be automatically provided to the medical transcriptionist associated with the user profile 18a in any suitable fashion. Other scenarios are also possible.

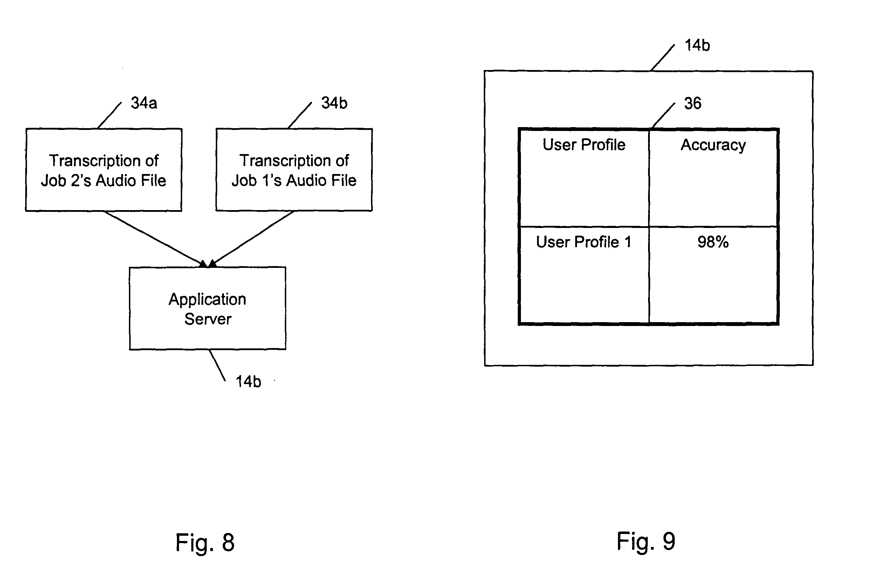

Referring now to Figure 8, the application server 14b receives transcriptions 34a, 34b of the audio files associated with the jobs 22a, 22b. The transcriptions 34a, 34b may be text files of any format and stored in any suitable fashion.

Quality personnel having access to the application server 14b, via the clients 12n illustrated in Figure 1, may access the transcriptions 34a, 34b and corresponding audio files 28a, 28b, and check the accuracy of the transcriptions 34a, 34b using known techniques. For example, the quality personnel may correct errors (grammar, spelling, missed words, etc.) in the transcriptions 34a, 34b and/or otherwise annotate the transcriptions 34a, 34b as necessary.

The application server 14b, based on the quality checks discussed above, may determine quality metrics associated with particular user profiles. As an example, in response to a selection of the user profile 18a, the application server 14b may determine a percentage accuracy of all of the transcriptions entered by the user profile 18a during a specified time period based on, for example, the number of corrections entered by the quality personnel. The number of corrected words divided by the total number of words in a particular transcription, for example, may yield a percentage accuracy for that transcription. The application server 14b may then average the percentage accuracies for all of user profile 18a's transcriptions. Other techniques, however, may also be used. Each correction may, for example, be assigned a weight less than 1 (e.g., 0.1, 0.25, etc.). The sum of these weights (for all corrections in one or more transcriptions) may be divided by the total number of lines (in the one or more transcriptions). This quotient may be subtracted from 1 and multiplied by 100 to yield a percentage accuracy.

Referring now to Figure 9, the application server 14b may report (display, print, etc.) the accuracy information determined as described above.

In other embodiments, in response to a specified percentage accuracy, the application server 14b may determine those user profiles that are either above or below the specified accuracy. For example, if the specified accuracy is 99%, the system may identify the user profile 18a as having an average accuracy less than 99% (in the example of Figure 9, the user profile 18a has an average accuracy of 98%). These features may be used to track the performance and progress of the various user profiles.

As apparent to those of ordinary skill, the algorithms disclosed herein may be deliverable to a processing device in many forms including, but not limited to, (i) information permanently stored on non-writable storage media such as ROM devices and (ii) information alterably stored on writeable storage media such as floppy disks, magnetic tapes, CDs, RAM devices, and other magnetic and optical media. The algorithms may also be implemented in a software executable object. Alternatively, the algorithms may be embodied in whole or in part using suitable hardware components, such as Application Specific Integrated Circuits (ASICs), state machines, controllers or other hardware components or devices, or a combination of hardware, software and firmware components.

While embodiments of the invention have been illustrated and described, it is not intended that these embodiments illustrate and describe all possible forms of the invention. The words used in the specification are words of description rather than limitation, and it is understood that various changes may be made without departing from the spirit and scope of the invention.

## Claims

1. A system for transcribing medical information comprising:
one or more computers configured to
receive input assigning each of a plurality of user profiles to at least one of a plurality of work pools based on user profile criteria,
receive a plurality of jobs each including demographic information, a priority indicator, and a digital audio file of patient medical information,
assign each of the jobs to one of the plurality of work pools based on the job's priority indicator and demographic information,
distribute each of the assigned jobs to one of the assigned user profiles active within the assigned job's work pool based on the assigned job's priority indicator,
receive, for each of the assigned jobs, a transcription of the job's digital audio file input by a user associated with the job's assigned user profile,
generate, for a selected user profile, quality metrics indicative of an accuracy of transcriptions input by the user associated with the selected user profile, and
output a visual representation of the quality metrics.

2. The system of claim 1 wherein the one or more computers are further configured to receive a specified transcription accuracy and to generate a list of user profiles based on the specified accuracy.

3. The system of claim 2 wherein the list includes user profiles having a transcription accuracy less than the specified accuracy.

4. The system of claim 2 wherein the list includes user profiles having a transcription accuracy greater than the specified accuracy.

5. The system of claim 1 wherein the priority indicator specifies a job turn-around time.

6. The system of claim 1 wherein the one or more computers are further configured to create a record in a database for each of the jobs, each record including a job identifier, the job's demographic information, and a stored location of the job's digital audio file.

7. A method for transcribing medical information comprising:
receiving input assigning, at one or more computers, each of a plurality of user profiles to at least one of a plurality of work pools based on user profile criteria;
receiving a plurality of jobs each including demographic information, a priority indicator, and a digital audio file of patient medical information;
assigning each of the jobs to one of the plurality of work pools based on the job's priority indicator and demographic information;
distributing each of the assigned jobs to one of the assigned user profiles active within the assigned
job's work pool based on the assigned job's priority indicator;
receiving, for each of the assigned jobs, a transcription of the job's digital audio file input by a user associated with the job's assigned user profile;
generating, for a selected user profile, quality metrics indicative of an accuracy of transcriptions input by the user associated with the selected user profile; and
outputting a visual representation of the quality metrics.

8. The method of claim 7 further comprising receiving a specified transcription accuracy and generating a list of user profiles based on the specified accuracy.

9. The method claim 8 wherein the list includes user profiles having a transcription accuracy less than the specified accuracy.

10. The method of claim 8 wherein the list includes user profiles having a transcription accuracy greater than the specified accuracy.

11. The method of claim 7 wherein the priority indicator specifies a job turn-around time.

12. The method of claim 7 further comprising creating a record in a database for each of the jobs, each record including a job identifier, the job's demographic information, and a stored location of the job's digital audio file.

13. A computer-readable storage medium having information stored thereon for directing one or more computers to (i) receive input assigning each of a plurality of user profiles to at least one of a plurality of work pools based on user profile criteria, (ii) receive a plurality of jobs each including demographic information, a priority indicator, and a digital audio file of patient medical information, (iii) assign each of the jobs to one of the plurality of work pools based on the job's priority indicator and demographic information, (iv) distribute each of the assigned jobs to one of the assigned user profiles active within the assigned job's work pool based on the assigned job's priority indicator, (v) receive, for each of the assigned jobs, a transcription of the job's digital audio file input by a user associated with the job's assigned user profile, (vi) generate, for a selected user profile, quality metrics indicative of an accuracy of transcriptions input by the user associated with the selected user profile, and (vii) output a visual representation of the quality metrics.
